Europäisches Patentamt

(19) European Patent Office

Office européen des brevets

(11) EP 0 685 180 B1

(12) **FASCICULE DE BREVET EUROPEEN**

(45) Date de publication et mention
de la délivrance du brevet:
**08.09.1999 Bulletin 1999/36**

(51) Int. Cl.$^6$: **A45D 19/02**

(21) Numéro de dépôt: **95401031.0**

(22) Date de dépôt: **04.05.1995**

(54) **Procédé de décoloration des cheveux par irradiation laser avec refroidissement, et dispositif pour sa mise en oeuvre**

Verfahren zum Bleichen von Haaren durch Laserbestrahlung mit Kühlung, und Vorrichtung zur Durchführung des Verfahrens

Process for bleaching hair by laser irradiation with cooling, and device for carrying out this process

(84) Etats contractants désignés:
**AT BE CH DE ES FR GB IT LI NL SE**

(30) Priorité: **04.05.1994 FR 9405468**

(43) Date de publication de la demande:
**06.12.1995 Bulletin 1995/49**

(73) Titulaire: **L'OREAL**
**75008 Paris (FR)**

(72) Inventeurs:
• **Caisey, Laurence**
**F-94400 Vitry-Sur-Seine (FR)**
• **Bauer, Daniel**
**F-93340 Le Raincy (FR)**
• **Sturla, Jean-Michel**
**F-92210 Saint-Cloud (FR)**

(74) Mandataire:
**Tonnellier, Jean-Claude**
**Nony & Associés,**
**29, rue Cambacérès**
**75008 Paris (FR)**

(56) Documents cités:
**WO-A-91/06279        US-A- 4 792 341**

• **LASER FOCUS. INCL. V NO. 2450 ELECTRO-OPTICS, SINCE 1983, vol. 19, no. 9, Septembre 1983 TULSA US, 'LASERS AND HAIR'**

## Description

L'invention concerne la décoloration des cheveux. Plus précisément, l'invention a pour objet un procédé de décoloration des cheveux par irradiation avec un rayonnement laser, ainsi qu'un dispositif pour mettre en oeuvre ce procédé.

On sait que pour décolorer ou éclaircir les cheveux, on utilise classiquement un traitement chimique à l'aide d'un agent oxydant, tel que le peroxyde d'hydrogène ou des persels, qui détruit au moins une partie des produits colorants naturels et/ou artificiels présents dans les cheveux.

La méthode classique de décoloration chimique des cheveux nécessite l'emploi d'agents oxydants relativement puissants et/ou concentrés, qui ont pour effet de dégrader non seulement les substances colorantes, mais aussi la fibre kératinique des cheveux. Il en résulte que les cheveux ainsi décolorés sont fragiles et doivent ensuite être traités avec précaution.

Pour ces raisons, il est impossible d'appliquer à de tels cheveux divers traitements cosmétiques tels que par exemple les traitements de déformation permanente utilisés pour les cheveux naturels, car ces traitements consistent à appliquer sur les cheveux un agent réducteur, à des pH relativement élevés, puis un agent oxydant, et ces agents ont des effets nettement agressifs sur la fibre kératinique des cheveux. En fait, l'application d'un traitement classique de déformation permanente sur une chevelure décolorée par voie chimique provoque des dégradations irréversibles et même des cassures des cheveux.

Cela explique que les coiffeurs expérimentés n'acceptent pas d'effectuer une opération de déformation permanente immédiatement ou peu de temps après une décoloration par voie chimique.

Par ailleurs, on sait qu'une proportion importante de personnes souhaite obtenir une chevelure dite "méchée", c'est-à-dire une chevelure qui n'est pas totalement décolorée, mais dans laquelle seulement certaines mèches de cheveux sont décolorées. Il s'agit d'une opération dite de "méchage". Le cas des chevelures méchées est particulièrement délicat, car on retrouve alors sur une même chevelure des cheveux naturels et des cheveux très décolorés.

La présente invention permet de remédier à ces divers inconvénients, grâce à un procédé de décoloration des cheveux par irradiation à l'aide d'un faisceau laser, dans des conditions particulières qui permettent d'obtenir, sur tous types de cheveux, colorés naturellement ou artificiellement, une décoloration d'intensité facilement contrôlable, sans dégradation notable de la fibre kératinique, et le traitement peut être assez rapide. Ce procédé est particulièrement bien adapté à l'obtention d'une chevelure méchée. En outre, les cheveux ainsi décolorés conservent les propriétés mécaniques et physico-chimiques qu'ils avaient avant la décoloration. Ils peuvent par exemple être soumis sans délai à un traitement de permanente, avec les compositions de permanente classiques.

La possibilité théorique de décolorer des cheveux avec un rayonnement laser a été mentionnée dans la publication Tech. News, *Laser Focus*, Vol. 19, No. 9, p.26, Septembre 1983. En outre, dans le brevet US-4 792 341, on a décrit un procédé de décoloration des cheveux par irradiation à l'aide d'un laser, ainsi qu'un dispositif expérimental permettant de déterminer la densité d'énergie, par unité de surface, qui permet de détruire la mélanine des cheveux à l'aide d'un rayonnement laser. En fait, ce brevet US ne décrit ni un procédé ni un dispositif permettant, en pratique, l'application de l'irradiation laser à la décoloration des cheveux de clients d'un salon de coiffure.

On a maintenant découvert que, de façon surprenante, il est possible de décolorer des mèches de cheveux, quelle que soit leur couleur d'origine (naturelle ou artificielle), sans dégradation de la fibre kératinique, en soumettant successivement des parties des mèches de cheveux à traiter à une irradiation par un faisceau laser émis sous forme d'impulsions à une fréquence de récurrence des impulsions suffisamment élevée pour ne pas allonger excessivement la durée du traitement, à condition de refroidir suffisamment la mèche de cheveux dans la zone traitée.

Des études sur cheveux isolés de diverses origines (cheveux naturels européens, japonais, mexicains et scandinaves) ont permis d'étudier la puissance lumineuse nécessaire pour obtenir une bonne décoloration des cheveux sans dégradation de la fibre kératinique, avec une seule impulsion laser (tir monocoup). On a constaté que les cheveux très foncés (japonais et mexicains) ne se décolorent pas suffisamment en profondeur ou éclatent sous l'irradiation lorsqu'on augmente encore la puissance par unité de surface, pour une durée d'impulsion donnée. En utilisant une puissance crête inférieure à la puissance crête la plus élevée qui, en tir monocoup, pour la durée d'impulsion utilisée et pour le type de cheveux étudié, ne provoque pas l'éclatement de la fibre kératinique, on a ensuite effectué des tirs successifs, en séquences (fréquence de récurrence des impulsions : 10 Hz), sur une même zone des cheveux isolés traités. On a découvert que les cheveux isolés, même les cheveux très foncés, pouvaient être décolorés sans dommage en abaissant ainsi la puissance crête, en effectuant plusieurs passages successifs sur une même zone traitée, ce qui permet de décolorer d'abord les couches superficielles, puis les couches plus profondes et finalement les cheveux peuvent être décolorés totalement.

Des études analogues faites sur des mèches de cheveux ont permis de constater que par irradiation en séquence sur un même endroit pendant 30 secondes, avec une fréquence de récurrence des impulsions de 10 Hz, les cheveux de la mèche sont fondus ou brûlés avant d'être décolorés, dans des conditions où l'on n'observait pas de dégradation dans le cas de cheveux isolés.

On a toutefois découvert qu'il est possible d'obtenir une décoloration sur tous types de cheveux rassemblés en mèches, et même à des fréquences d'impulsions supérieures à 10 Hz, à condition de refroidir la partie traitée de la mèche de cheveux, par exemple à l'aide d'un flux gazeux localisé.

Ainsi, une irradiation laser de puissance crête suffisante, émise sous forme d'impulsions en séquences, et combinée à un refroidissement de la mèche de cheveux dans la partie irradiée, permet une décoloration homogène, en profondeur, sur toute la longueur des mèches si désiré, et sur tous types de cheveux, sans dégradation des cheveux malgré une puissance moyenne d'irradiation relativement élevée, et avec des durées de traitement raisonnables. Des résultats analogues peuvent aussi être obtenus avec des cheveux teints artificiellement : il faut alors en général augmenter la puissance du faisceau laser, et ici encore le refroidissement s'impose.

Comme cela apparaîtra évident pour les spécialistes, c'est en fait l'énergie fournie aux grains de mélanine, sur une durée de temps suffisamment courte, qui doit être suffisante pour dégrader ou détruire la mélanine. C'est donc en réalité la densité d'énergie fournie par unité de surface, en un temps suffisamment court, qui doit atteindre un seuil suffisamment élevé pour que les cheveux puissent être décolorés. Dans la présente demande, lorsqu'on parle de "puissance" ou de "puissance crête", il faut comprendre qu'il s'agit d'une simplification de langage, car c'est en fait l'énergie fournie lors de chaque impulsion qui est importante, et il faut donc tenir compte de la durée de l'impulsion, qui doit être toutefois non supérieure à une microseconde environ (durée approximative du temps de relaxation de la mélanine) dans le cas de la décoloration des cheveux naturels.

On a également découvert qu'il est possible de décolorer de façon analogue les cheveux colorés artificiellement, à condition d'opérer, comme pour les cheveux non teints, en adaptant la puissance du rayonnement laser à la couleur naturelle des cheveux. Ce n'est qu'après avoir effectué cette étape préliminaire, correspondant à la dégradation de la mélanine, que l'on peut entreprendre la décoloration proprement dite, correspondant à la dégradation du colorant artificiel. En effet, la dégradation des colorants artificiels nécessite des énergies plus importantes que la dégradation de la mélanine, de sorte que si l'on cherchait à dégrader directement lesdits colorants, les cheveux seraient détruits par éclatement de la fibre kératinique, comme dans les expériences rapportées ci-dessus.

On sait par ailleurs que certaines personnes âgées ont une chevelure "blanche" qui a en fait une teinte jaunâtre peu esthétique. Le procédé de l'invention permet de transformer cette coloration jaunâtre en blanc pur.

L'invention a donc pour objet un procédé de traitement de décoloration au moins partielle d'au moins une mèche ou partie de mèche de cheveux, ce procédé étant tel que défini dans la revendication 1.

On sait qu'un laser se compose essentiellement d'un milieu actif rendu amplificateur par un mécanisme de pompage fournissant de l'énergie aux atomes de façon sélective, ledit milieu actif étant enfermé dans une cavité résonnante. Le milieu actif est alors capable d'émettre un faisceau lumineux, sensiblement monochromatique, polarisé et cohérent. En raison de cette cohérence, un faisceau laser concentre une énergie nettement plus importante que celle d'un rayonnement émis par une source lumineuse classique.

Certains lasers, notamment ceux à milieu actif solide, sont capables d'émettre un rayonnement laser sous la forme d'impulsions très brèves (généralement entre la femtoseconde et la microseconde). La concentration de l'énergie sur des intervalles de temps aussi brefs confère à l'impulsion laser une puissance, dite puissance crête, considérable. On utilise de préférence dans le procédé de l'invention des lasers permettant la production d'impulsions contrôlées. On peut utiliser par exemple des lasers à rubis ou des lasers dont le milieu actif contient des ions de terres rares ou d'actinides, par exemple un laser à néodyme. La réalisation de tels lasers est bien connue. Les ions actifs peuvent être insérés dans une matrice cristalline telle que le grenat d'yttrium-aluminium (en abrégé YAG), ou dans une matrice amorphe telle qu'un verre. On règle la fréquence de récurrence des impulsions à l'aide d'une lampe de pompage à éclairs. On peut régler l'énergie disponible à l'aide de systèmes classiques, notamment des polariseurs.

On utilise de préférence des lasers émettant dans l'ultraviolet proche, dans le visible ou dans le proche infrarouge, par exemple à une longueur d'onde pouvant aller de 300 à 1100 nm. On peut utiliser par exemple un laser YAG-néodyme qui émet à 1,06 $\mu$m éventuellement, avec un multiplicateur de fréquence qui permet par exemple d'obtenir une émission de longueur d'ondes 532 nm (fréquence double) ou de 355 nm (fréquence triple).

La détermination du couple puissance crête-durée d'impulsion (par exemple la puissance crête du faisceau laser pour une durée d'impulsion donnée) peut être effectuée dans chaque cas par de simples expériences de routine.

La puissance crête doit être suffisante pour décolorer la mélanine et éventuellement le colorant artificiel. La puissance crête doit être inférieure à la puissance qui endommage le cheveu considéré. Pour décolorer la mélanine, on peut utiliser par exemple une puissance crête fournissant, par impulsion, une densité d'énergie pouvant aller de 0,1 à 1,2 J/cm$^2$ environ, selon la couleur naturelle et l'origine des cheveux, même s'il s'agit de cheveux teints. Plus précisément, il est souhaitable de ne pas dépasser une densité d'énergie maximum par impulsion qui est de l'ordre de :

- 0,35 J/cm$^2$ pour les cheveux brun foncé (de type japonais ou mexicain),
- 0,4 J/cm$^2$ pour les cheveux châtain foncé,
- 0,5 J/cm$^2$ pour les cheveux châtain clair,
- 0,7 J/cm$^2$ pour les cheveux blond foncé,
- 1,2 J/cm$^2$ pour les cheveux blond clair.

Les diverses données fournies ci-dessus concernant la densité d'énergie ont été établies pour un rayonnement de longueur d'onde 532 nm. Lorsque la longueur d'onde utilisée est différente, il convient d'appliquer un facteur de correction

$$\frac{\lambda \text{ nm}}{532}$$

comme cela est exposé plus en détail dans la partie expérimentale ci-après.

Par ailleurs, on rappelle que les différentes teintes de cheveux peuvent être définies de façon objective par la luminance (L), selon le système de coordonnées colorimétriques C.I.E.(L,a,b). Dans la présente demande, les teintes des cheveux mentionnées correspondent aux gammes de luminance mentionnées ci-après :

| Type de cheveux | L |
|---|---|
| japonais ou mexicain | inférieure à 18 |
| châtain foncé | 18-20 |
| châtain clair | 22-24 |
| blond foncé | 28-35 |
| blond clair | 45-52 |

Le procédé décrit ci-dessus, qui correspond à une décoloration au moins partielle par dégradation de la mélanine des cheveux, doit être effectué dans tous les cas, même si l'on traite des cheveux teints à l'aide d'un agent colorant, et dans ce dernier cas, le procédé de décoloration comporte une étape supplémentaire d'irradiation des cheveux, analogue à celle décrite ci-dessus, mais par un rayonnement laser fournissant une densité d'énergie plus élevée, suffisante pour détruire ou dégrader l'agent colorant ; cette densité d'énergie est notamment au moins égale à 0,8 J/cm$^2$. Elle est généralement inférieure à 2 J/cm$^2$ à 532 nm.

Pour mettre en oeuvre l'étape préliminaire de dégradation de la mélanine, il suffit de connaître la couleur naturelle des cheveux teints à traiter, et on applique alors des conditions d'irradiation convenant pour les cheveux ayant cette couleur naturelle, lesdites conditions ayant été déterminées préalablement, une fois pour toutes, par de simples expériences de routine.

La puissance crête maximum que peuvent supporter les cheveux dépend de leur couleur. Comme indiqué ci-dessus, si la puissance crête est trop élevée, la fibre kératinique éclate.

Il est donc possible d'opérer entre une puissance crête minimum et une puissance crête maximum, qui dépend du type de cheveux. La fréquence des impulsions peut aller par exemple jusqu'à 1000 Hz, notamment de 10 à 100 Hz environ. La capacité du système de refroidissement à évacuer la chaleur produite, de façon à éviter la dégradation thermique des cheveux, doit évidemment être adaptée au choix de la fréquence des impulsions, qui elle-même, conditionne la durée du traitement.

La durée des impulsions peut aller par exemple de 10 picosecondes à 100 nanosecondes.

Le procédé de l'invention nécessite évidemment de décolorer la chevelure par mèches, en irradiant successivement des zones de ladite mèche. Généralement, la surface d'irradiation peut varier dans la gamme de 0,1-2 cm$^2$.

Le procédé de l'invention est donc particulièrement bien adapté à l'obtention de chevelures méchées. Pour traiter successivement les zones à décolorer par déplacement relatif d'une mèche par rapport au faisceau laser, on peut déplacer la mèche par rapport à l'appareil utilisé pour le traitement, ou vice-versa, et/ou faire varier périodiquement la direction du faisceau laser de façon à effectuer des balayages successifs de la zone traitée. Cette variation périodique de la direction du faisceau laser peut être obtenue par exemple à l'aide d'un miroir oscillant.

On peut refroidir les cheveux dans la zone traitée notamment par circulation d'un fluide. La solution la plus simple est de créer un flux gazeux baignant les cheveux dans la zone traitée, par exemple par aspiration de l'air ou encore en soufflant un gaz, éventuellement refroidi au préalable, tel que de l'air, de l'azote, de l'hélium, du dioxyde de carbone, etc. On peut bien entendu utiliser un gaz chargé de vapeur d'eau ou chargé de particules liquides (aérosol), par exemple des gouttelettes d'eau.

Les cheveux traités peuvent être secs ou humides.

Pour pouvoir décolorer la mèche de cheveux traitée dans de bonnes conditions, il est préférable de disposer cette mèche sous la forme d'une nappe de cheveux, et on peut alors décolorer les cheveux de la zone traitée en irradiant au moins une des faces de ladite nappe. De préférence, l'épaisseur de la nappe de cheveux correspond à l'épaisseur de 3 à 20 cheveux superposés environ.

Selon un mode de réalisation particulier, le procédé de l'invention peut encore présenter les caractéristiques suivantes :

- on opère à l'aide d'un dispositif de traitement comprenant un réceptacle pour la mèche de cheveux à traiter, la surface dudit réceptacle comportant un orifice pour la sortie dudit faisceau laser,
- on applique, sur au moins une partie de leur longueur, les cheveux de ladite mèche étalés en nappe sur la surface dudit réceptacle, de façon qu'une zone à traiter soit en regard dudit orifice,
- on procède à l'irradiation de ladite zone,
- et, par déplacements relatifs de ladite mèche par rapport au faisceau laser, on procède successivement à l'irradiation des autres zones à traiter. On décrira ci-après des dispositifs permettant une telle mise en oeuvre. Avec ces dispositifs, les déplacements relatifs de la mèche par rapport au faisceau laser peuvent être obtenus par des déplacements relatifs de la mèche par rapport audit réceptacle et/ou par variations périodiques de la direction du faisceau laser, comme indiqué précédemment.

L'un des avantages du procédé de décoloration des cheveux par irradiation avec un faisceau laser est qu'il est possible de contrôler en continu la décoloration produite et d'arrêter le traitement au degré de décoloration choisi.

Le réglage des moyens de refroidissement, par exemple le débit du flux gazeux destiné à refroidir les cheveux dans la zone traitée, peut être déterminé préalablement par de simples expériences de routine, en fonction de la puissance moyenne dissipée. On peut aussi effectuer cette détermination dans chaque cas en procédant à des essais préalables sur un échantillon des cheveux à traiter.

Comme indiqué ci-dessus, l'avantage principal de la décoloration par irradiation laser est que les cheveux ne sont pas dégradés et conservent les propriétés mécaniques et physico-chimiques qu'ils avaient avant traitement. Il s'agit là d'un avantage considérable puisque l'on peut effectuer sur les cheveux ainsi décolorés tous autres traitements cosmétiques sans prendre de précautions particulières.

L'invention a également pour objet un dispositif pour la mise en oeuvre d'un procédé de décoloration des cheveux tel que défini précédemment. Ce dispositif comprend :

- un corps muni d'un réceptacle, ledit réceptacle étant destiné à servir de logement, sur au moins une partie de la longueur des cheveux, pour une mèche de cheveux à traiter étalée en nappe, et
- des moyens destinés à acheminer un faisceau laser de façon à irradier au moins une zone d'une mèche de cheveux disposée dans ledit réceptacle, et est caractérisé par le fait qu'il comprend en outre des moyens pour refroidir lesdits cheveux dans la zone ou les zones d'irradiation.

Dans des modes de réalisation particuliers, le dispositif de l'invention peut encore présenter les caractéristiques suivantes, prises isolément ou, le cas échéant, en combinaison :

- lesdits moyens pour refroidir les cheveux comprennent des moyens pour créer un flux gazeux, à l'aide d'un conduit relié à une source de gaz comprimé ou à un système d'aspiration;
- ledit réceptacle comprend une rainure à fond large, permettant d'étaler ladite nappe sur ledit fond;
- ladite rainure peut être associée à une pièce dégageable de forme coopérante capable d'insertion dans ladite rainure en ménageant, entre ladite pièce et le fond de la rainure, un espace constituant un logement pour ladite nappe; ladite pièce est par exemple dégageable par actionnement d'un levier permettant d'introduire la mèche à traiter dans le réceptacle ou de l'en retirer; le fond de ladite rainure et/ou une face de ladite pièce en regard dudit fond peut comporter un orifice pour la sortie d'un faisceau laser vers la zone à irradier; de même, le fond de ladite rainure ou une face de ladite pièce en regard dudit fond peut comporter un orifice pour la création d'un flux gazeux (par aspiration ou par soufflage);
- dans un autre mode de réalisation, ledit réceptacle comprend une rainure en forme de fente fine débouchant à la surface dudit corps et ayant une profondeur suffisante pour permettre l'insertion de ladite nappe sur la totalité de sa largeur; de façon analogue à celle mentionnée ci-dessus pour le premier mode de réalisation, l'une au moins des faces en regard de ladite fente fine peut comporter un orifice pour la sortie d'un faisceau laser; et l'une au moins des faces en regard de ladite fente fine peut comporter un orifice pour la création d'un flux gazeux.

Il faut noter enfin que le canal laser et le conduit de création d'un flux gazeux peuvent être confondus.

Bien entendu, le réceptacle doit constituer un espace confiné étanche à la lumière pour éviter que le rayonnement laser puisse provoquer des dommages à l'utilisateur ou à sa clientèle.

On va maintenant illustrer l'invention en faisant référence aux dessins annexés dans lesquels :

- la figure 1 est une vue schématique d'un premier mode de réalisation du dispositif de l'invention, qui comprend à son extrémité un réceptacle sous forme de rainure à fond large et une pièce coopérante actionnable par un levier,
- la figure 2 est une vue partielle de dessus de l'extrémité, portant le réceptacle, du dispositif de la figure 1,
- la figure 3 est une vue partielle de dessous de l'extrémité de ladite pièce coopérante du dispositif de la figure 1,
- la figure 4 illustre schématiquement le mode d'utilisation du dispositif de la figure 1,
- la figure 5 représente une vue schématique en coupe d'un second mode de réalisation du dispositif, avec un réceptacle en forme de fente fine,
- la figure 6 est une vue en coupe longitudinale verticale du dispositif de la figure 5,
- et la figure 7 schématise le mode d'utilisation du dispositif de la figure 5.

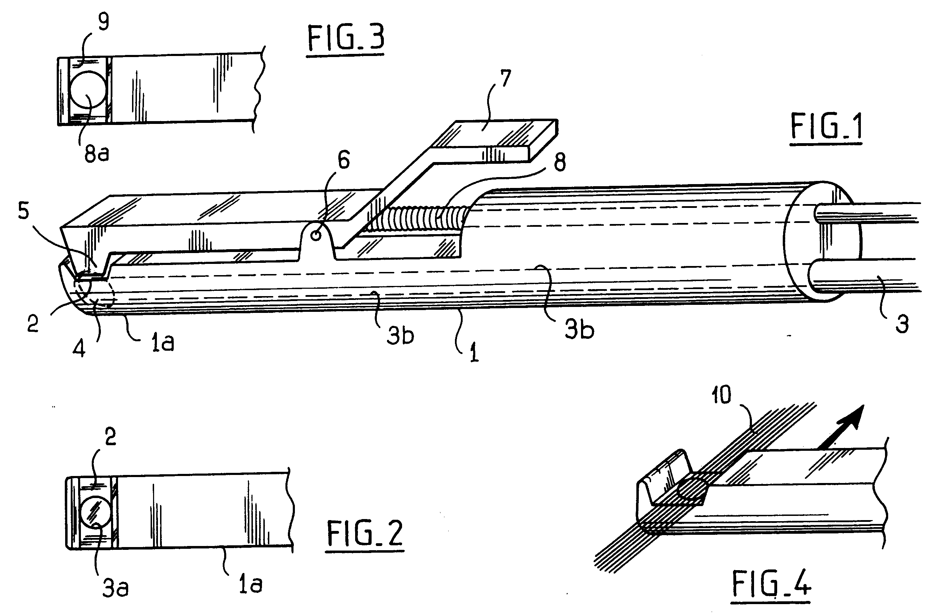

On voit sur la figure 1 que le dispositif comprend un corps allongé (1) partiellement creux dont l'extrémité antérieure (1a) de section réduite, comporte un réceptacle ayant la forme d'une rainure à fond plat (2). Un conduit (3) pour le faisceau laser débouche dans le fond de la rainure (2) après renvoi coudé à l'aide du miroir (4), et forme une lucarne de sortie (3a) à la surface de la paroi (2). Les pointillés (3b) schématisent la propagation du faisceau laser à l'intérieur du corps (1) ou peuvent représenter un guide de lumière.

Une pièce coopérante (5) mobile autour de l'axe (6) est maintenue engagée dans le réceptacle à l'aide de moyens de rappel tels qu'un ressort à tension (non représenté) et est dégageable par actionnement du levier (7) dont est munie la pièce (5) à son extrémité arrière. Un tube souple (8) est relié à une source d'air comprimé (non représenté) et débouche dans la face (9), en regard du fond de la rainure, de la pièce (5), par un orifice (8a). La partie arrière du corps (1) sert de poignée de prise en main de l'appareil par le manipulateur. Pour décolorer les cheveux, on dégage la pièce (5) pour pouvoir disposer la mèche de cheveux (10), répartie en nappe comme indiqué à la figure 4, puis on laisse la pièce (5) se réengager dans la rainure. La mèche se trouve donc disposée dans l'espace entre les faces (2) et (9), on actionne l'émetteur laser (non représenté) ainsi que le système de refroidissement et on déplace le dispositif par rapport à la mèche (ou vice-versa) pour décolorer successivement toute la mèche ou une partie de mèche à traiter. Il est préférable d'effectuer des déplacements qui ne soient pas trop lents, par exemple de l'ordre de 0,1-5 centimètres par seconde, et d'opérer par passages successifs, ce qui permet de contrôler très facilement l'évolution de la décoloration, dans de bonnes conditions de sécurité. Mais il est également possible, avec un refroidissement efficace, d'utiliser des vitesses de déplacement plus faibles, ou des déplacements discontinus.

Le corps du dispositif peut être réalisé en tout matériau approprié, par exemple en métal léger comme l'aluminium ou en matière plastique. Le tuyau (8) est un tuyau souple classique. Il peut être relié à un système d'aspiration à la place de la source d'air comprimé.

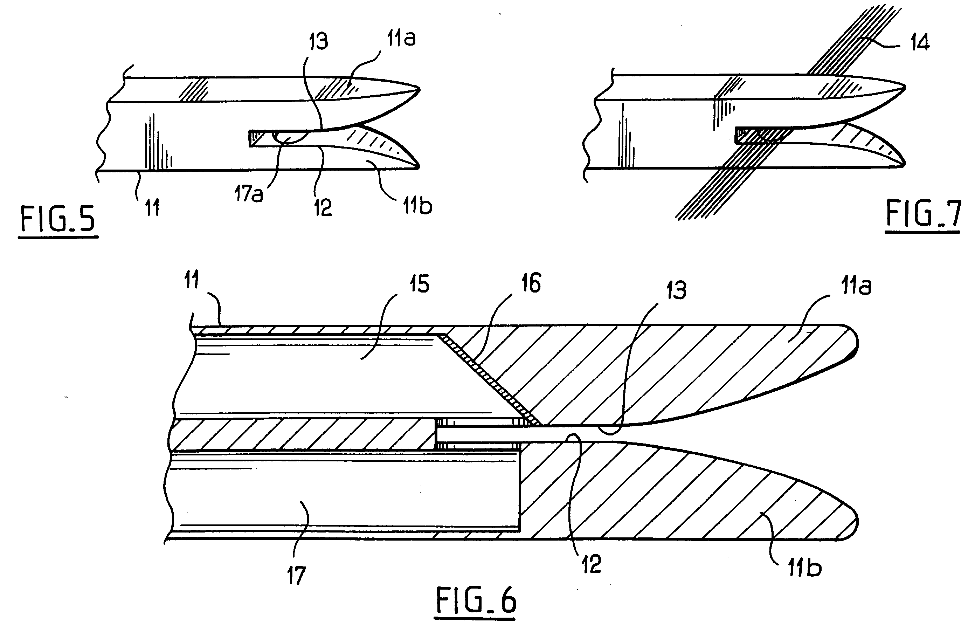

Le dispositif représenté aux figures 5 et 6 comporte un corps allongé (11) dont on n'a représenté que la partie antérieure, la partie arrière non représentée formant poignée. On voit que l'extrémité du corps (11) se sépare en deux branches (11a) et (11b) séparées par une fente dont les faces en regard (12) et (13) constituent un logement pour une mèche de cheveux (14) disposée comme indiqué à la figure 7. Comme dans le premier mode de réalisation, un canal laser (15) débouche sur la face (13) de la fente après renvoi par le miroir (16), et un canal (17) relié à une source d'air comprimé débouche à la surface de la paroi (12) en (17a).

On utilise ce dispositif de façon analogue au précédent. Les organes de commande du faisceau laser et du circuit de refroidissement, qui peuvent être couplés, peuvent être installés sur le dispositif ou dans un organe de commande séparé qui peut être éventuellement actionné au pied. On peut également prévoir des dispositif d'asservissement de la puissance laser, en fonction de la couleur de la mèche à traiter, cet asservissement pouvant être réalisé de façon automatique après lecture de la couleur par un détecteur approprié.

Les exemples suivants illustrent l'invention.

**EXEMPLE 1 :**

Dans cet exemple et dans les exemples suivants, on utilise des mèches de cheveux de 0,25 g, ayant une longueur de 20 cm.

L'appareil utilisé est un appareil du type représenté à la figure 1.

La source de rayonnement laser est un laser Surelite Continuum ; longueur d'onde : 532 nm ; fréquence des tirs : 1 Hz ; diamètre du faisceau : 5 mm ; durée de l'impulsion ; 4 ns.

Avec cet appareillage, on a étudié les plages de décoloration optimale, selon la couleur des cheveux à traiter.

Les gammes d'énergie par $cm^2$ pour une impulsion, sont celles qui sont utilisables pour décolorer effectivement les cheveux.

En-dessous de la valeur minimum, il n'y a pas de décoloration notable. Au-dessus de la valeur maximum, la fibre du cheveu éclate ou est fissurée (les dommages sont visibles, selon leur importance, à la loupe binoculaire, au microscope ou au microscope électronique).

Les résultats sont résumés dans le tableau (I) suivant :

TABLEAU (I)

| Cheveux | énergie/$cm^2$ pour 1 impulsion (en J/$cm^2$) |
|---|---|
| japonais | 0,2 à 0,35 |
| châtain foncé | 0,2 à 0,4 |
| châtain clair | 0,15 à 0,5 |
| blond foncé | 0,15 à 0,7 |
| blond clair | 0,1 à 1,2 |

Par ailleurs, l'absorption de l'énergie lumineuse de la mélanine varie avec la longueur d'onde : elle diminue lorsque la longueur d'onde augmente, de sorte que les cheveux supportent sans dégradation une densité d'énergie incidente plus élevée lorsque la longueur d'onde augmente. L'étude expérimentale a montré que la densité d'énergie maximale supportable par les cheveux sans éclatement de la fibre kératinique, pour un rayonnement de longueur d'onde $\lambda$, est sensiblement celle indiquée dans le tableau ci-dessus, multipliée par un facteur

$$\frac{\lambda}{532}$$

où $\lambda$ est exprimée en nanomètres. Cette loi de variation avec la longueur d'onde vaut également pour la relation entre la densité d'énergie incidente et l'efficacité de la décoloration : la densité d'énergie capable de décolorer les cheveux d'un type donné, pour la longueur d'onde $\lambda$, est sensiblement égale à la densité d'énergie permettant d'obtenir une décoloration analogue avec un rayonnement de longueur d'onde 532 nm, multipliée par ledit facteur

$$\frac{\lambda}{532}$$

Pour des cheveux colorés artificiellement, il faut utiliser des densités d'énergie assez élevées, généralement au moins égales à 0,8 J/cm$^2$ par impulsion. Si les cheveux ont été colorés sans décoloration préalable, il faut tenir compte de leur teinte naturelle. Par exemple, si la teinte naturelle des cheveux était châtain clair, il faut d'abord utiliser une densité d'énergie non supérieure à 0,5 J/cm$^2$ (voir tableau 1 ci-dessus) pour décolorer la mélanine. Ce n'est qu'ensuite qu'on pourra utiliser une densité d'énergie supérieure (1 J/cm$^2$ ou plus) pour détruire le colorant artificiel. Si l'on appliquait dès le début cette densité d'énergie élevée, les cheveux éclateraient.

**EXEMPLE 2 :**

On opère comme à l'exemple 1, avec un laser Quanta Ray Spectra Physics ; longueur d'onde : 532 nm ; fréquence des tirs : 50 Hz ; diamètre du faisceau : 8 mm ; durée de l'impulsion : 7 ns.
Le refroidissement est assuré par un débit d'air de 0,5 litre par seconde.
Avec cet appareil, la puissance crête par unité de surface correspondant à une décoloration optimale pour des cheveux châtain foncé est de l'ordre de 40 MW/cm$^2$, soit une énergie par unité de surface de 0,3 J/cm$^2$.

**EXEMPLE 3 :**

On opère comme précédemment, avec un laser BMI présentant les caractéristiques suivantes : longueur d'onde : 523 nm ; fréquence des tirs : 20 Hz ;

diamètre du faisceau : 3 mm ; durée de l'impulsion : 30 ps ; refroidissement par flux d'hélium.
On a obtenu une décoloration optimale de cheveux châtain foncé avec une énergie par unité de surface de 0,28 J/cm$^2$.
Si on opère en coupant le débit d'hélium, on observe une dégradation du cheveu avec fusion des écailles, visible à la loupe ou au microscope.

**EXEMPLE 4 :**

Avec l'appareillage décrit à l'exemple 1, on a décoloré des mèches de cheveux de 0,2 g, longueur 20 cm, en déplaçant lentement la pince à décolorer le long de la mèche.
Refroidissement par air ; débit 0,25 litre par seconde.
Fréquence des impulsions : 10 Hz.
Avec des cheveux châtain foncé et un passage de la totalité de la mèche en 5 minutes environ, on a obtenu une décoloration complète après 5 passages, en utilisant une puissance crête correspondant à une énergie de 0,35 J/cm$^2$ par impulsion.
Pour des cheveux blond clair, on a obtenu une décoloration complète après un seul passage d'une durée d'une minute environ.
Avec des cheveux châtain foncé, on a fait des mesures de solubilité alcaline sur les cheveux naturels, les cheveux décolorés par rayonnement laser et les cheveux décolorés par voie chimique (peroxyde d'hydrogène).
La solubilité alcaline sert à caractériser l'état de dégradation des cheveux. Les mèches sont immergées pendant 30 minutes à 65°C dans une solution 0,1 N d'hydroxyde de sodium, puis rincées 3 fois par immersion pendant 5 minutes dans de l'eau distillée, et enfin séchées à l'étuve à 105°C jusqu'à poids constant. La perte de poids des cheveux, en %, représente la solubilité alcaline.
On a également procédé à une détermination de l'acide cystéique. Après un traitement d'hydrolyse des cheveux à chaud en milieu acide, on mesure la quantité d'acide cystéique passée en solution, en séparant les acides aminés sur résine échangeuse d'ions et en effectuant une réaction colorée à la ninhydrine. Sur cheveux naturels, le taux d'acide cystéique est entre 0 et 0,8 %. Sur cheveux dégradés, ce taux augmente.
On a constaté que la décoloration laser ne modifie pratiquement pas la solubilité alcaline, ni la teneur en acide cystéique alors qu'elles sont fortement augmentées après décoloration par voie chimique.

**Revendications**

1. Procédé de traitement de décoloration au moins partielle d'au moins une mèche ou partie de mèche de cheveux, par irradiation de ladite mèche ou partie de mèche à l'aide d'un rayonnement laser de puissance suffisante pour décolorer les cheveux, dans lequel :

- on traite une zone de ladite partie de mèches par irradiation à l'aide d'un faisceau laser émis sous forme d'impulsions, pour décolorer au moins partiellement les cheveux de ladite zone par dégradation de la mélanine des cheveux,
- le cas échéant, par déplacement relatif de ladite mèche par rapport audit faisceau laser, on traite successivement, de façon analogue, une ou plusieurs autres zones de façon à traiter la totalité de ladite partie de mèche,
- on répète éventuellement les traitements précédents jusqu'à obtention du degré de décoloration souhaité pour ladite mèche ou partie de mèche,
  caractérisé par le fait que ladite irradiation sous forme d'impulsions est effectuée à une fréquence d'impulsions d'au moins 10 Hz et que, pendant ledit traitement de décoloration, on refroidit les cheveux, dans la zone traitée, de façon suffisante pour éviter un échauffement local dommageable pour les cheveux.

2. Procédé selon la revendication 1, caractérisé par le fait que l'on refroidit les cheveux, dans la zone traitée, par circulation d'un fluide.

3. Procédé selon la revendication 2, caractérisé par le fait que l'on refroidit les cheveux par création d'un flux gazeux baignant les cheveux dans la zone traitée.

4. Procédé selon l'une quelconque des revendications précédentes, caractérisé par le fait que l'on opère avec un rayonnement laser fournissant une densité d'énergie par impulsion qui n'est pas supérieure à un seuil au-delà duquel la fibre kératinique serait endommagée, ledit seuil étant d'autant plus faible que la couleur naturelle des cheveux à traiter est plus foncée.

5. Procédé selon la revendication 4, caractérisé par le fait que ladite densité d'énergie est de 0,1-1,2 J/cm$^2$ à 532 nm.

6. Procédé selon l'une quelconque des revendications précédentes, caractérisé par le fait que l'on opère de façon à ne pas dépasser une densité d'énergie maximum par impulsion qui est de l'ordre de :

- 0,35 J/cm$^2$ pour les cheveux de type japonais,
- 0,4 J/cm$^2$ pour les cheveux châtain foncé,
- 0,5 J/cm$^2$ pour les cheveux châtain clair,
- 0,7 J/cm$^2$ pour les cheveux blond foncé,
- 1,2 J/cm$^2$ pour les cheveux blond clair,

les valeurs de densité d'énergie étant données ici pour un rayonnement de longueur d'onde de 532 nm, et devant être multipliées par un facteur de correction égal à :

$$\frac{\lambda}{532}$$

lorsque le rayonnement utilisé a une longueur d'onde λ (exprimée en nanomètres) autre que 532 nm.

7. Procédé selon l'une quelconque des revendications précédentes, caractérisé par le fait que l'on opère de façon à obtenir une densité d'énergie (en joules/cm$^2$), par impulsion, de :

- 0,2-0,35 pour les cheveux de type japonais,
- 0,2-0,4 pour les cheveux châtain foncé,
- 0,15-0,5 pour les cheveux châtain clair,
- 0,15-0,7 pour les cheveux blond foncé,
- 0,1-1,2 pour les cheveux blond clair,

les valeurs de densité d'énergie étant données ici pour un rayonnement de longueur d'onde de 532 nm, et devant être multipliées par un facteur de correction égal à :

$$\frac{\lambda}{532}$$

lorsque le rayonnement utilisé a une longueur d'onde λ (exprimée en nanomètres) autre que 532 nm.

8. Procédé selon l'une quelconque des revendications précédentes, caractérisé par le fait qu'en outre, lorsque les cheveux à traiter sont teints à l'aide d'un agent colorant, on effectue une étape supplémentaire d'irradiation des cheveux, de façon analogue à celle décrite dans l'une quelconque des revendications précédentes, mais avec une densité d'énergie plus élevée, suffisante pour détruire ou dégrader l'agent colorant.

9. Procédé selon la revendication précédente, caractérisé par le fait que ladite densité d'énergie plus élevée est au moins égale à 0,8 J/cm$^2$ par impulsion à 532 nm.

10. Procédé selon l'une quelconque des revendications précédentes, caractérisé par le fait que l'on dispose préalablement ladite mèche de façon à former une nappe de cheveux, et que l'on décolore les cheveux de la zone traitée en irradiant au moins une des faces de ladite nappe.

11. Procédé selon l'une quelconque des revendications précédentes, caractérisé par le fait :

- que l'on opère à l'aide d'un dispositif de traitement comprenant un réceptacle pour la mèche de cheveux à traiter, la surface dudit réceptacle comportant un orifice pour la sortie dudit faisceau laser,
- que l'on applique, sur au moins une partie de leur longueur, les cheveux de ladite mèche étalés en nappe sur la surface dudit réceptacle, de façon qu'une zone à traiter soit en regard dudit orifice,
- que l'on procède à l'irradiation de ladite zone,
- et que par déplacements relatifs de ladite mèche par rapport au faisceau laser, on procède successivement à l'irradiation des autres zones à traiter.

12. Procédé selon la revendication précédente, caractérisé par le fait que lesdits déplacements relatifs comprennent des déplacements relatifs de ladite mèche par rapport audit réceptacle.

13. Procédé selon l'une quelconque des revendications 11 et 12, caractérisé par le fait que lesdits déplacements relatifs sont obtenus en faisant varier la direction du faisceau laser de façon à effectuer des balayages de la zone à traiter.

14. Dispositif pour la mise en oeuvre d'un procédé tel que défini dans l'une quelconque des revendications précédentes, comprenant:

- un corps (1) muni d'un réceptacle (2), ledit réceptacle étant destiné à servir de logement, sur au moins une partie de la longueur des cheveux, pour une mèche de cheveux à traiter étalée en nappe, et
- des moyens (3, 3b, 4, 3a) destinés à acheminer un faisceau laser de façon à irradier au moins une zone d'une mèche de cheveux disposée dans ledit réceptacle, caractérisé par le fait qu'il comprend en outre des moyens (8, 8a) pour refroidir lesdits cheveux dans la zone ou les zones d'irradiation.

15. Dispositif selon la revendication 14, caractérisé par le fait que lesdits moyens pour refroidir les cheveux comprennent des moyens pour créer un flux gazeux.

16. Dispositif selon la revendication 14 ou 15, caractérisé par le fait que ledit réceptacle comprend une rainure à fond large (2), permettant d'étaler ladite nappe sur ledit fond.

17. Dispositif selon la revendication 16, caractérisé par le fait qu'à ladite rainure est associée une pièce dégageable (5) de forme coopérante capable d'insertion dans ladite rainure en ménageant, entre ladite pièce et le fond de la rainure, un espace constituant un logement pour ladite nappe.

18. Dispositif selon la revendication 17, caractérisé par le fait que ladite pièce est dégageable par actionnement d'un levier (7) permettant d'introduire la mèche à traiter dans le réceptacle ou de l'en retirer.

19. Dispositif selon l'une quelconque des revendications 16 à 18, caractérisé par le fait que le fond de ladite rainure et/ou une face de ladite pièce en regard dudit fond comporte un orifice pour la sortie d'un faisceau laser vers la zone à irradier.

20. Dispositif selon l'une quelconque des revendications 16 à 19, caractérisé par le fait que le fond de ladite rainure ou une face de ladite pièce en regard dudit fond comporte un orifice (8a) pour la création d'un flux gazeux.

21. Dispositif selon la revendication 14, caractérisé par le fait que ledit réceptacle comprend une rainure en forme de fente fine débouchant à la surface dudit corps et ayant une profondeur suffisante pour permettre l'insertion de ladite nappe sur la totalité de sa largeur.

22. Dispositif selon la revendication 21, caractérisé par le fait que l'une au moins des faces en regard (12,13) de ladite fente fine comporte un orifice pour la sortie d'un faisceau laser.

23. Dispositif selon l'une quelconque des revendications 21 et 22, caractérisé par le fait que l'une au moins des faces en regard (12,13) de ladite fente fine comporte un orifice pour la création d'un flux gazeux.

**Claims**

1. Process for bleaching at least one lock or portion of lock of hair, at least partially, by irradiation of the said lock or portion of lock using a laser beam of sufficient power to bleach the hair, in which:

- an area of the said portion of lock is treated by irradiation using a laser beam emitted in the form of pulses, in order to bleach, at least partially, the hair in the said area, by degradation of the melanin in the hair,
- if required, by relative movement of the said lock with respect to the said laser beam, one or more other areas are treated in succession, in a similar way, so as to treat the totality of the said portion of lock,
- the above treatments are possibly repeated until the desired degree of bleaching is obtained for the said lock or portion of lock,

characterized in that the said irradiation in the form of pulses is carried out at a pulse frequency of at least 10 Hz and that, during the said bleaching treatment, the hair is cooled, in the treated area, sufficiently to prevent local overheating which can damage the hair.

2. Process according to Claim 1, characterized in that the hair is cooled, in the treated area, by circulation of a fluid.

3. Process according to Claim 2, characterized in that the hair is cooled by creating a gas flow bathing the hair in the treated area.

4. Process according to any one of the preceding claims, characterized in that the operation is performed with laser radiation delivering, per pulse, an energy density which is not greater than a threshold above which the keratinous fibre of the hair would be damaged, the said threshold being lower the darker the natural colour of the hair to be treated.

5. Process according to Claim 4, characterized in that the said energy density is from 0.1 - 1.2 J/cm$^2$ at 532 nm.

6. Process according to any one of the preceding claims, characterized in that the operation is performed so as not to exceed a maximum energy density per pulse which is of the order of:

   - 0.35 J/cm$^2$ for hair of the Japanese type,
   - 0.4 J/cm$^2$ for dark chestnut hair,
   - 0.5 J/cm$^2$ for light chestnut hair,
   - 0.7 J/cm$^2$ for dark blond hair,
   - 1.2 J/cm$^2$ for light blond hair,

   the values of energy density being given here for radiation of 532 nm wavelength and having to be multiplied by a correction factor equal to:

   $$\frac{\lambda}{532}$$

   when the radiation used has a wavelength $\lambda$ (expressed in nanometres) other than 532 nm.

7. Process according to any one of the preceding claims, characterized in that the operation is performed so as to obtain an energy density (in joules/cm$^2$), per pulse, of:

   - 0.2 - 0.35 for Japanese-type hair,
   - 0.2 - 0.4 for dark chestnut hair,
   - 0.15 - 0.5 for light chestnut hair,
   - 0.15 - 0.7 for dark blond hair,
   - 0.1 - 1.2 for light blond hair,

   the values of energy density being given here for radiation of 532 nm wavelength and having to be multiplied by a correction factor equal to:

   $$\frac{\lambda}{532}$$

   when the radiation used has a wavelength $\lambda$ (expressed in nanometres) other than 532 nm.

8. Process according to any one of the preceding claims, characterized in that, furthermore, during the bleaching step, when the hair to be treated is dyed using a colouring agent, an additional step of irradiation of the hair is performed, in a way similar to that described in any one of the preceding claims, but with a higher energy density, sufficient to destroy or degrade the colouring agent.

9. Process according to the preceding claim, characterized in that the said higher energy density is at least equal to 0.8 J/cm$^2$ per pulse at 532 nm.

10. Process according to any one of the preceding claims, characterized in that, during the bleaching step, the said lock is arranged beforehand so as to form a ribbon of hair and in that the hair in the treated area is bleached by irradiating at least one of the faces of the said ribbon.

11. Process according to any one of the preceding claims, characterized in that:

    - the operation is performed using a treatment device which includes a receptacle for the lock of hair to be treated, the surface of the said receptacle having an orifice for the exit of the said laser beam,
    - the hair of the said lock, spread out in the form of a ribbon, is applied, over at least part of its length, to the surface of the said receptacle so that an area to be treated is opposite the said orifice,
    - irradiation of the said area is then carried out,
    - and, by relative movements of the said lock with respect to the laser beam, irradiation of the other areas to be treated is carried out in succession.

12. Process according to the preceding claim, characterized in that the said relative movements include relative movements of the said lock with respect to the said receptacle.

13. Process according to either of Claims 11 and 12, characterized in that the said relative movements are obtained by varying the direction of the laser beam so as to perform scanning of the area to be treated.

14. Device for implementing a process as defined in any one of the preceding claims, including:

   - a body (1) equipped with a receptacle (2), the said receptacle being intended to serve as a housing, over at least part of the length of the hair, for a lock of hair to be treated, the lock being spread out in the form of a ribbon, and
   - means (3, 3b, 4, 3a) intended to convey a laser beam so as to irradiate at least one area of a lock of hair arranged in the said receptacle; characterized in that it also includes means (8, 8a) for cooling said hair in the irradiation area or areas.

15. Device according to Claim 14, characterized in that the said means for cooling the hair comprise means for creating a gas flow.

16. Device according to Claim 14 or 15, characterized in that the said receptacle includes a wide-bottomed groove (2), making it possible to spread out the said ribbon on the said bottom.

17. Device according to Claim 16, characterized in that the said groove is associated with a disengageable piece (5) of cooperating shape, capable of being inserted into the said groove, leaving, between the said piece and the bottom of the groove, a space forming a housing for the said ribbon.

18. Device according to Claim 17, characterized in that the said piece can be disengaged by actuating a lever (7) which makes it possible to introduce the lock to be treated into the receptacle or to remove it therefrom.

19. Device according to any one of Claims 16 to 18, characterized in that the bottom of the said groove and/or one face of the said piece opposite the said bottom has an orifice for the exit of a laser beam onto the area to be irradiated.

20. Device according to any one of Claims 16 to 19, characterized in that the bottom of the said groove or one face of the said piece opposite the said bottom has an orifice (8a) for creating a gas flow.

21. Device according to Claim 14, characterized in that the said receptacle includes a groove in the form of a narrow slot emerging at the surface of the said body and having a depth sufficient to allow insertion of the said ribbon over the totality of its width.

22. Device according to Claim 21, characterized in that at least one of the faces (12, 13) opposite the said narrow slot has an orifice for the exit of a laser beam.

23. Device according to any one of Claims 21 or 22, characterized in that at least one of the faces (12, 13) opposite the said narrow slot has an orifice for creating a gas flow.

## Patentansprüche

1. Behandlungsverfahren zur mindestens teilweisen Entfärbung mindestens einer Haarsträhne oder eines Teils einer Haarsträhne durch Bestrahlung der Strähne oder eines Teils hiervon mit Hilfe von Laserstrahlung genügender Stärke um die Haare zu entfärben, bei dem man

   - eine Zone eines Teils der Strähne durch Bestrahlung mit Hilfe eines emittierten Impulslasers behandelt, um mindestens teilweise die Haare dieser Zone durch Abbau des Melanins der Haare zu entfärben,

   - gegebenenfalls durch relative Verlagerung der Strähne in Bezug auf den Laserstrahl, auf gleiche Weise eine oder mehrere andere Zonen nacheinander so behandelt, daß der gesamte Teil der Strähne behandelt wird,

   - man gegebenenfalls die vorhergehenden Behandlungen wiederholt bis man den gewünschten Entfärbungsgrad der Strähne oder des Teils der Strähne erzielt,

   - dadurch gekennzeichnet, daß man die Impulsionsstrahlung bei einer Impulsfrequenz von mindestens 10 Hz durchführt, und daß man während der Entfärbungsbehandlung die Haare in der behandelten Zone abkühlt, um eine lokale, für die Haare schädliche Erhitzung zu vermeiden.

2. Verfahren nach Anspruch 1, dadurch gekennzeichnet, daß man die Haare in der behandelten Zone durch Zirkulation eines Fluids abkühlt.

3. Verfahren nach Anspruch 2, dadurch gekennzeichnet, daß man die Haare durch Erzeugung eines Gasstroms abkühlt, der die Haare in der behandelten Zone umfließt.

4. Verfahren nach einem der vorhergehenden Ansprüche, dadurch gekennzeichnet, daß man mit einer Laserstrahlung arbeitet, die eine Dichte der Impulsenergie ergibt, die nicht oberhalb einer Schwelle liegt, jenseits der die Keratinfaser beschädigt würden, wobei diese Schwelle um so niedriger liegt, je dunkler die natürliche Farbe der zu behandelten Haare ist.

5. Verfahren nach Anspruch 4, dadurch gekennzeichnet, daß die Energiedichte 0,1-1,2 J/cm$^2$ bei 532 nm ist.

6. Verfahren nach einem der vorgehenden Ansprüche, dadurch gekennzeichnet, daß eine maximale Energiedichte des Impulses nicht überschritten wird, in der Größenordnung von:

- 0,35 J/cm$^2$ für Haare des japanischen Typs,
- 0,4 J/cm$^2$ für dunkelbraunes Haar,
- 0,5 J/cm$^2$ für hellbraunes Haar,
- 0,7 J/cm$^2$ für dunkelblondes Haar,
- 1,2 J/cm$^2$ für hellblondes Haar,

wobei die hier wiedergegebenen Werte der Energiedichte für eine Strahlung der Wellenlänge von 532 nm gelten und mit einem Korrekturfaktor:

$$\frac{\lambda}{532}$$

multipliziert werden müssen, wenn die verwendete Strahlung eine andere Wellenlänge $\lambda$ (ausgedrückt in Nanometer) als 532 nm aufweist.

7. Verfahren nach einem der vorhergehenden Ansprüche, dadurch gekennzeichnet, daß man auf eine Weise arbeitet, daß man eine Dichte der im Impulsenergie (in Joules/cm$^2$) erhält von:

- 0,2-0,35 J/cm$^2$ für Haare des japanischen Typs,
- 0,2-0,4 J/cm$^2$ für dunkelbraunes Haar,
- 0,15-0,5 J/cm$^2$ für hellbraunes Haar,
- 0,15-0,7 J/cm$^2$ für dunkelblondes Haar,
- 0,1-1,2 J/cm$^2$ für hellblondes Haar,

wobei die hier gegebenen Wette der Energiedichte für eine Wellenlänge von 532 nm gelten, und durch einen Korrekturfaktor:

$$\frac{\lambda}{532}$$

multipliziert werden müssen, wenn die verwendete Strahlung eine andere Wellenlänge $\lambda$ (ausgedrückt in Nanometer) als 532 nm aufweist.

8. Verfahren nach einem der vorhergehenden Ansprüche, dadurch gekennzeichnet, daß man außerdem, wenn das zu behandelnde Haar mit Hilfe eines Farbstoffes gefärbt ist, eine zusätzliche Strahlungsstufe des Haares durchführt, auf gleiche Weise wie in einem der vorhergehenden Ansprüche beschrieben, jedoch mit einer höheren Energiedichte, die ausreicht, um den Farbstoff zu zerstören oder abzubauen.

9. Verfahren nach dem vorhergehenden Anspruch, dadurch gekennzeichnet, daß die höhere Energiedichte mindestens gleich 0,8 J/cm$^2$ pro Impuls bei 532 nm ist.

10. Verfahren nach einem der vorhergehenden Ansprüche, dadurch gekennzeichnet, daß man die Strähne vorher so anordnet, daß sie eine Haarfläche bildet und man die Haare der behandelten Zone entfärbt, in dem man mindestens eine Seite der Fläche bestrahlt.

11. Verfahren nach einem der vorgehenden Ansprüche, dadurch gekennzeichnet, daß man

- mit Hilfe einer Behandlungsvorrichtung arbeitet, die ein Aufnahmegefäß für die zu behandelnde Haarsträhne aufweist, wobei die Oberfläche des Aufnahmegefäßes eine Öffnung für den Austritt des Laserstrahls aufweist,
- mindestens auf einem Teil ihrer Länge die Haare der als Fläche ausgebreiten Strähne auf die Oberfläche des Aufnahmegefäßes in der Weise aufbringt, daß eine zu behandelnde Zone gegenüber der Öffnung liegt,
- mit der Bestrahlung dieser Zone fortschreitet,
- durch relative Verschiebung der Strähne in Bezug auf den Lasserstrahl man nacheinander mit der Bestrahlung der anderen zu behandelnden Zonen fortschreitet.

12. Verfahren nach dem vorhergehenden Anspruch, dadurch gekennzeichnet, daß die relativen Verschiebungen relativ der Strähne in Bezug auf das Gefäß stattfinden.

13. Verfahren nach einem der Ansprüche 11 und 12, dadurch gekennzeichnet, daß die relativen Verschiebungen dadurch erhalten werden, daß man die Richtung des Lasserstrahls auf die Weise variiert, daß man die zu behandelnde Zone überstreicht.

14. Vorrichtung zur Durchführung eines Verfahrens nach einem der vorhergehenden Ansprüche umfaßend:

- ein mit einem Aufnahmegefäß (2) ausgerüstetes Gehäuse (1), wobei das Aufnahmegefäß zur Aufnahme mindestens eines Teils der Länge der Haare bestimmt ist, für eine als Fläche ausgebildeten Haarsträhne,

- Mittel (3, 3b, 4, 3a), die dazu bestimmt sind, einen Laserstrahl auf die Weise zu leiten, daß mindestens eine Zone der Haarsträhne in dem Aufnahmegefäß bestrahlt wird, dadurch gekennzeichnet, daß sie außerdem Mittel (8, 8a) für die Kühlung der Haare in der Zone oder den Zonen der Bestrahlung aufweist.

15. Vorrichtung nach Anspruch (14), dadurch gekennzeichnet, daß die Mittel zur Kühlung der Haare Mittel zur Schaffung eines Gasstromes umfassen.

16. Vorrichtung nach Anspruch 14 oder 15, dadurch gekennzeichnet, daß das Aufnahmegefäß eine Aussparung mit breiter Vertiefung (2) umfaßt, die es erlaubt, die Fläche auf dem Boden auszubreiten.

17. Vorrichtung nach Anspruch 16, dadurch gekennzeichnet, daß die Aussparung mit einem abnehmbaren Stück (5) in kooperierender Weise befähigt zur Insertion in die Aussparung beim Anlegen verbunden ist, daß zwischen dem Stück und dem Boden ein Raum besteht, der eine Lagerung der Fläche bildet.

18. Vorrichtung nach Anspruch 17, dadurch gekennzeichnet, daß das Stück durch Betätigung eines Hebels (7) beweglich ist, was es gestattet, die zu behandelnde Strähne in das Aufnahmegefäß einzuführen oder daraus zu entfernen.

19. Vorrichtung nach einem der Ansprüche 16 bis 18, dadurch gekennzeichnet, daß der Boden der Aussparung und/oder eine Seite des Stückes in Bezug auf den Boden eine Öffnung für den Ausgang eines Laserstrahls gegen die zu bestahlende Zone aufweist.

20. Vorrichtung nach einem der Ansprüche 16 bis 19, dadurch gekennzeichnet, daß der Boden der Aussparung oder eine Seite des Stückes gegenüber dem Boden eine Öffnung (8a) für die Schaffung eines Gasstromes aufweist.

21. Vorrichtung nach Anspruch 14, dadurch gekennzeichnet, daß das Aufnahmegefäß eine Aussparung in Form einer feinen Spalte aufweist, die auf der Oberfläche des Gehäuses verläuft und eine ausreichende Tiefe hat, um die Einführung der Fläche über ihre ganze Länge zu erlauben.

22. Vorrichtung nach Anspruch 21, dadurch gekennzeichnet, daß das mindestens eine Seite gegenüber (12, 13) der feinen Spalte eine Öffnung für den Austritt eines Laserstrahls aufweist.

23. Vorrichtung nach einem der Ansprüche 21 und 22 dadurch gekennzeichnet, daß mindestens eine Seite gegenüber (12, 13) der feinen Spalte eine Öffnung für die Schaffung eines Gasstromes aufweist.

FIG.3

FIG.1

FIG.2

FIG.4

EP 0 685 180 B1

FIG.5

FIG.7

FIG.6

EP 0 685 180 B1